(19) **Europäisches Patentamt / European Patent Office / Office européen des brevets**

(11) **EP 2 258 253 B1**

(12) **EUROPEAN PATENT SPECIFICATION**

(45) Date of publication and mention of the grant of the patent:
**07.05.2014 Bulletin 2014/19**

(51) Int Cl.:
*A61B 1/06* (2006.01)     *A61B 5/00* (2006.01)

(21) Application number: **10164730.3**

(22) Date of filing: **02.06.2010**

(54) **Endoscopic apparatus and endoscopic image obtainment method**

Endoskopische Vorrichtung und Verfahren zum Erhalt eines endoskopischen Bildes

Appareil endoscopique et procédé d'obtention d'image endoscopique

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **04.06.2009 JP 2009134680**

(43) Date of publication of application:
**08.12.2010 Bulletin 2010/49**

(73) Proprietor: **FUJIFILM Corporation
Minato-ku
Tokyo (JP)**

(72) Inventors:
• **Kubo, Masahiro
Kanagawa-ken (JP)**

• **Ohta, Yasunori
Kanagawa-ken (JP)**
• **Okoyama, Kazuo
Kanagawa-ken (JP)**

(74) Representative: **Klunker . Schmitt-Nilson . Hirsch
Patentanwälte
Destouchesstrasse 68
80796 München (DE)**

(56) References cited:
**JP-A- 2007 264 537     US-A1- 2009 023 991
US-A1- 2009 137 908**

**Description**

BACKGROUND OF THE INVENTION

Field of the Invention

The present invention relates to an endoscopic apparatus that obtain spectral estimation images by matrix operation and an endoscopic image obtainment method.

Description of the Related Art

Endoscopic apparatuses are used to diagnose the conditions of the body cavities of patients, such as esophagi, stomachs, and large intestines, based on endoscopic images of the patients. The endoscopic apparatuses obtain the images of the body cavities of patients by inserting scopes into the body cavities. In recent years, when diagnosis is performed by using an endoscope, endoscopic images obtained by a scope are displayed on a monitor in real time. Therefore, a doctor checks imaged positions or regions by looking at the monitor, and diagnoses the patient based on the images (image diagnosis). Further, observation by endoscopes includes an ordinary observation mode, a fluorescent observation mode, a narrow-band mode, and the like to facilitate image diagnosis by doctors. The ordinary observation mode observes the subject illuminated with white light. The fluorescent observation mode observes fluorescence output from the subject when the subject is illuminated with fluorescence. The narrow-band mode observes the subject illuminated with narrow band light.

Further, as light sources for endoscopes, use of LED's (light emitting diodes) instead of tungsten or the like has been proposed (for example, please refer to Japanese Unexamined Patent Publication No. 2006-166940 (Patent Document 1)). An endoscopic apparatus disclosed in Patent Document 1 includes four LED light sources that have different wavelength bands from each other. Further, Patent Document 1 discloses the feature that the LED's and control operations are switched based on an ordinary observation mode, a fluorescent observation mode, and an NBI mode.

Further, generation of spectral images by operation processing based on image signals obtained by using white light has been proposed. The white light is used instead of narrow band light, which is used in a narrow band mode. In this method, relationships between numerical data representing the color sensitivity characteristic of each of R, G and B and numerical data representing the spectral characteristic of a specific narrow band-pass filter are obtained as matrix data (coefficient sets) . A spectral estimation image is obtained by estimating a spectral image that will be obtained through the narrow band-pass filter by performing operation using the matrix data and RGB signals. When the spectral image is generated by performing operation as described above, it is not necessary to prepare a plurality of filters corresponding to desirable wavelength bands. Further, it is not necessary to change or arrange the plurality of filters. Therefore, it is possible to prevent the size of the endoscopic apparatus from becoming large, and to lower the cost of the endoscopic apparatus.

Further, when spectral images are generated by matrix operation, changing the intensity of light output from an LED light source has been proposed (for example, please refer to Japanese Unexamined Patent Publication No. 2007-264537 (Patent Document 2)). Specifically, three LED's that have different wavelength bands from each other are prepared. When the wavelength band of a spectral image is selected, the intensity of light of each of the three LED's is changed, and a frame image is obtained for the wavelength band of each of the LED's. Further, a single spectral image is generated by using the three frame images.

However, when a spectral image is generated by using a plurality of frame images, as proposed in Patent Document 2, a time period corresponding to the plurality of frame images is required, and the time period for generating the spectral image becomes long. Therefore, the frame rate of video images (motion images, dynamic images or the like) becomes lower, and the image quality becomes lower. Hence, efficient image diagnosis becomes impossible.

JP 2007-264537 discloses an endoscopic apparatus in which a plurality of wavelength bands that constitute a spectral estimation image is set as a wavelength set. Three types of combined illuminating light beams are irradiated onto a target, wherein the light beams are obtained by variably adjusting the light output intensity of each of plural light emitting elements. Based on three frames of image data obtained by irradiation of each of the combined light beams, a spectral estimation image for the set wavelength set is generated.

SUMMARY OF THE INVENTION

In view of the foregoing circumstances, it is an object of the present invention to provide an endoscopic apparatus that can efficiently generate accurate spectral images, and an endoscopic image obtainment method.

An endoscopic apparatus of the present invention is an endoscopic apparatus comprising the features of claim 1.

An endoscopic image obtainment method of the present invention is an endoscopic image obtainment method comprising the steps of claim 4.

Here, the light source control means should control, based on the wavelength set, ON/OFF of the plurality of light emitting devices. When each of the wavelength bands included in the wavelength set overlaps with at least one of the wavelength bands of the plurality of light emitting devices, the light source control means may drive only the light emitting devices of the overlapping wavelength bands. When at least one of the wavelength bands included in the wavelength set overlaps with none of the wavelength bands of the plurality of light emitting devices, the light source control means may drive the plurality of light emitting devices of wavelength bands that are necessary to generate the spectral estimation image.

The light source unit may include the plurality of light emitting devices corresponding to respective wavelength bands included in the plurality of wavelength sets.

Further, when at least one of the wavelength bands included in the wavelength set overlaps with none of the wavelength bands of the plurality of light emitting devices, the light source control means may drive all of the plurality of light emitting devices. Alternatively, the light source control means may drive a combination of light emitting devices selected from the plurality of light emitting devices, the combination achieving highest estimation accuracy.

According to the endoscopic apparatus and the endoscopic image obtainment method of the present invention, a plurality of wavelength bands to be represented by a spectral estimation image are set as a wavelength set. Further, a light source unit including a plurality of light emitting devices that output light of different wavelength bands from each other, the light source unit being able to output white light by driving the plurality of light emitting devices, is controlled. The light source unit is controlled by controlling ON/OFF of each of the plurality of light emitting devices based on the set wavelength set. Further, a spectral estimation image is generated by performing, based on the set wavelength set, matrix operation on an endoscopic image that has been obtained by a scope while ON/OFF of each of the plurality of light emitting devices is controlled. Therefore, it is possible to improve the estimation accuracy of the spectral estimation image by outputting light of a wavelength band or bands that are necessary to generate the spectral estimation image from an endoscopic image. Further, it is possible to efficiently generate a spectral estimation image.

When the wavelength set setting means sets the wavelength set by selecting a wavelength set from a plurality of wavelength sets that have been prepared advance, and the light source unit includes a plurality of light emitting devices corresponding to respective wavelength bands included in the plurality of wavelength sets, if a user selects an arbitrary wavelength set, the light emitting devices are automatically drive-controlled based on the selection by the user. Therefore, it is possible to improve the estimation accuracy of the spectral estimation image and to efficiently generate a spectral estimation image.

Further, when at least one of the wavelength bands included in the wavelength set overlaps with none of the wavelength bands of the plurality of light emitting devices, the light source control means may drive a combination of light emitting devices selected from the plurality of light emitting devices, the combination achieving highest estimation accuracy. When the light source control means operates in such a manner, even if at least one of the wavelength bands included in the wavelength set overlaps with none of the wavelength bands of the plurality of light emitting devices, it is possible to maintain the estimation accuracy of the spectral estimation image at a high level.

BRIEF DESCRIPTION OF THE DRAWINGS

Figure 1 is a block diagram illustrating an endoscopic apparatus according to an embodiment of the present invention;
Figure 2 is a table showing an example of matrix parameters used by a spectral image generation means illustrated in Figure 1;
Figure 3••is a table showing an example of corresponding relationships between wavelength sets that are set by a wavelength set setting means illustrated in Figure 1 and ON/OFF control of each light emitting device; and
Figure 4 is a flow chart illustrating an endoscopic image obtainment method according to an embodiment of the present invention.

DESCRIPTION OF THE PREFERRED EMBODIMENTS

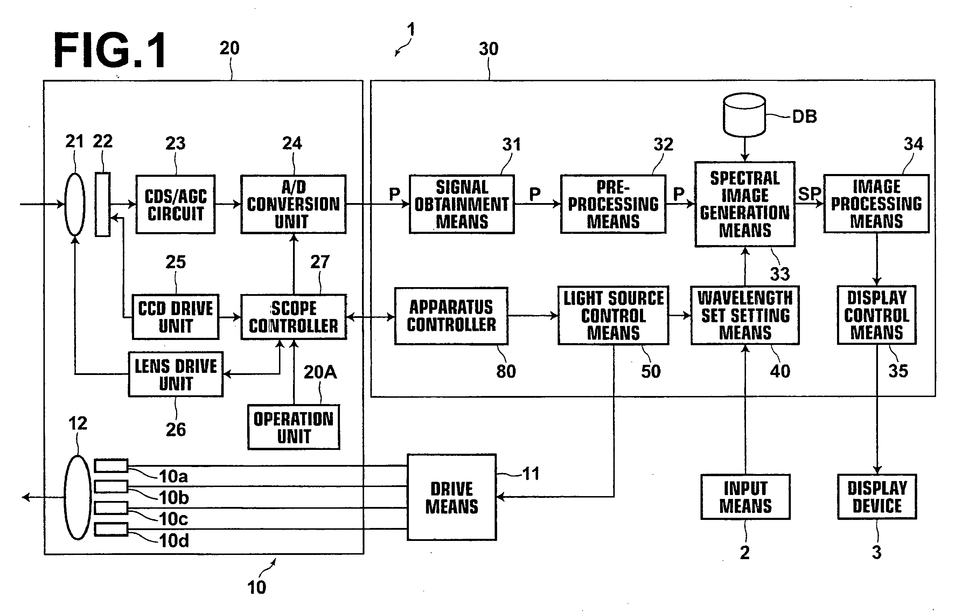

Hereinafter, embodiments of the present invention will be described in detail with reference to drawings. Figure 1 is a block diagram illustrating an example of an endoscopic apparatus according to the present invention. An endoscopic apparatus 1 includes a light source unit 10, a scope 20, and an endoscopic image processing apparatus 30. The light source unit 10 outputs light to a subject to observe the subject by an endoscope. The light source unit 10 includes a plurality of light emitting devices 10a through 10d that output light of different wavelength bands from each other. The plurality of light emitting devices 10a through 10d are arranged at the leading end of the scope 20. Specifically, the light emitting devices 10a through 10d include LED's the peak wavelengths of which are 470 nm, 500 nm, 540 nm, and 620 nm, respectively. Therefore, white light is output from the light source unit 10 when all of the light emitting devices 10a through 10d are driven or three light emitting devices, namely light emitting devices 10a, 10b (or 10c), and 10d, are driven. The plurality of light emitting devices 10a through 10d emit light by application of drive currents by a drive means 11. Further, the operation of the drive means 11 is controlled by a light source control means 50.

The scope 20 includes an imaging lens 21, an imaging device 22, a CDS/AGC circuit 23, an A/D converter 24, a CCD drive unit 25, a lens drive unit 26, and the like. Each element of the scope 20 is controlled by a scope controller 27. The imaging lens 21 is composed of a plurality of lens groups for example. The imaging magnification of the imaging lens 21 is changed by being driven by a lens drive unit 26. The imaging device 22 includes, for example, a CCD, a CMOS, or the like. The imaging device 22 obtains an image by performing photoelectric conversion on an image of the subject formed by the imaging lens 21. As the imaging device 22, a complementary color type device, or a primary color type device is used for example. The complementary color type device has a color filter of Mg (magenta), Ye (yellow), Cy (cyan), and G (green) on the imaging surface thereof. The primary color type device has a color filter of RGB. Further, the operation of the imaging device 22 is controlled by a CCD drive unit 25. When the imaging device 22 obtains image (video) signals, the CDS/AGC (correlated double sampling / automatic gain control) circuit 23 performs sampling on the image (video) signals, and amplifies the sampled signals. Further, the A/D converter 24 performs A/D conversion on an endoscopic image output from the CDS/AGC circuit 23, and outputs the converted endoscopic image to the endoscopic image processing apparatus 30.

The endoscopic image processing apparatus 30 processes endoscopic image P, obtained by using the scope 20. For example, the endoscopic image processing apparatus 30 is configured by a DSP (digital signal processor) or the like. The endoscopic image processing apparatus 30 includes an image obtainment means 31, a pre-processing means 32, a spectral image generation means 33, an image processing means, and a display control means 35. The image obtainment means 31 obtains endoscopic image P imaged by the imaging device 22 in the scope 20. The pre-processing means 32 performs pre-processing on the endoscopic image P obtained by the image obtainment means 31. For example, when the endoscopic image P is represented by a YCC color system, the pre-processing means 32 converts the endoscopic image P into an image represented by an RGB color system. Further, the pre-processing means 32 has a gamma conversion function (gamma correction function), a gradation adjustment function, and the like.

The spectral image generation means 33 generates spectral estimation image SP by performing matrix operation on the endoscopic image P by using matrix parameter M. An example of the operation by the spectral image generation means 33 is described in detail in Japanese Unexamined Patent Publication No. 2003-093336.

Specifically, the spectral image generation means 33 generates the spectral estimation image SP by performing matrix operation using the following formula (1):

[Formula 1]

$$
\begin{pmatrix} SP_r \\ SP_g \\ SP_b \end{pmatrix} = \begin{pmatrix} M_{00} & M_{01} & M_{02} \\ M_{10} & M_{11} & M_{12} \\ M_{20} & M_{21} & M_{22} \end{pmatrix} \cdot \begin{pmatrix} Pr \\ Pg \\ Pb \end{pmatrix} \qquad \cdots \quad (1)
$$

In Formula (1), $Sp_r$, $Sp_g$, $Sp_b$ represent R, G and B components of the spectral estimation image SP, respectively. Pr, Pg and Pb represent R, G and B components of the endoscopic image P, respectively. Values $M_{00}$ through $M_{22}$ in the matrix of $3 \times 3$ (three columns and three rows) represent matrix parameters M for matrix operation, respectively.

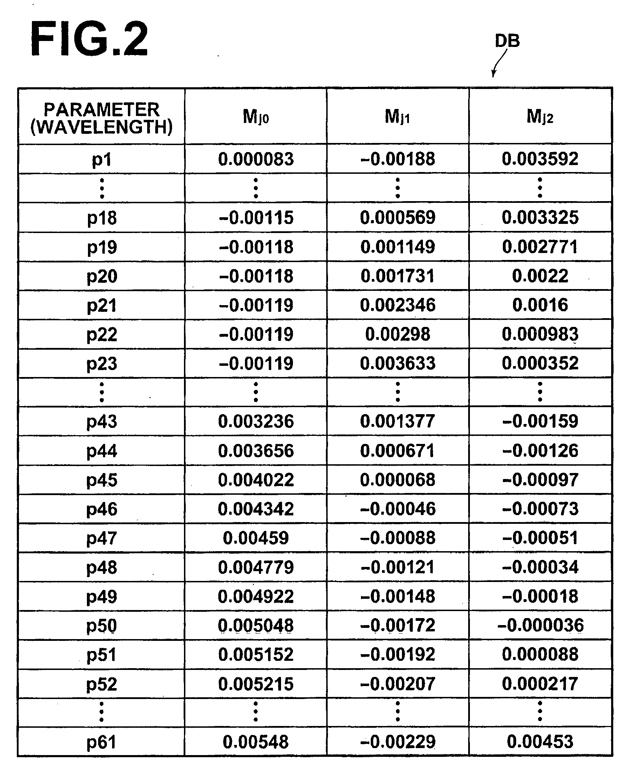

Figure 2 is a table showing an example of database DB that stores matrix parameters for performing the matrix operation represented by Formula (1). In Figure 2, the database DB stores parameter $pi = (M_{j0}, M_{j1}, M_{j2})$ (i is a sign for distinguishing parameter sets stored in the database DB from each other, and i = 1 through 61, and j represents the rows of matrix M in Formula (1), and j = 0 through 2) for each of 61 wavelength bands, into which the wavelength band of 400 nm to 700 nm is divided at 5 nm intervals.

The wavelength set setting means 40 illustrated in Figure 1 sets, as a wavelength set, a plurality of wavelength bands to be represented by spectral estimation image SP. For example, the wavelength set setting means 40 has, in advance, a plurality of wavelength sets that are appropriate to observe respective regions, such as blood vessels and a living body tissue. The wavelength set setting means 40 sets a wavelength set by selecting the wavelength set based on an input from an input means. Specifically, for example, three wavelength sets CH1 (550 nm, 500 nm, 470 nm), CH2 (525 nm, 495 nm, 495 nm), and CH3 (540 nm, 415 nm, 415 nm) are prepared as wavelength sets for setting matrix parameter M. Further, the wavelength set setting means 40 may have a function for setting, as the wavelength set for spectral estimation image SP, an arbitrary wavelength set based on an input by a user, instead of the wavelength set that has been prepared in advance.

An image processing means 34 illustrated in Figure 1 performs enhancement processing or the like on endoscopic image p and spectral estimation image SP. A display control means 35 has a function for displaying the endoscopic image P and the spectral estimation image SP on which image processing has been performed by the image processing means 34. The display control means 35 displays the images on a display device 3 together with character information or the like.

A light source control means 50 has a function for controlling the light emitting devices 10a through 10d based on the wavelength set that has been set by the wavelength set setting means 40. Specifically, first, the light source control means 50 judges whether each of all the wavelength bands included in the wavelength set that has been set by the wavelength set setting means 40 overlaps with (or is substantially the same as) at least one of the wavelength bands of the light emitting devices 10a through 10d.

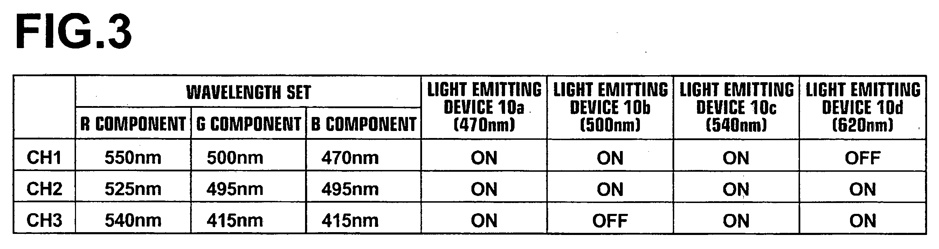

When the light source control means 50 judges that each of all the wavelength bands included in the wavelength set that has been set by the wavelength set setting means 40 overlaps with at least one of the wavelength bands of the light emitting devices 10a through 10d, the light source control means 50 drives only the light emitting device or devices the wavelength band or bands of which overlap. For example, as illustrated in Figure 3, when wavelength set CH1 including, as components of spectral estimation image SP, R component of 550 nm, G component of 500 nm, and B component of 470 nm is set, the wavelength bands included in the wavelength set are substantially the same as the wavelength bands of the light emitting devices 10a, 10b and 10c of the light emitting devices 10a through 10d. Therefore, the light source control means 50 controls the light emitting device 10d to OFF state, and the light emitting devices 10a, 10b, and 10c to ON state so that the endoscopic image P is obtained while the light emitting devices 10a, 10b and 10c are emitting light.

In contrast, when at least one of the wavelength bands included in the wavelength set overlaps with none of the wavelength bands of the light emitting devices, the light source control means 50 drives the light emitting devices 10a through 10d of wavelength bands that are necessary to generate the spectral estimation image SP. Specifically, when the wavelength bands included in the wavelength set overlap with none of the wavelength bands of the light emitting devices 10a through 10d, as in wavelength set CH2 in Figure 3, the light source control means 50 drives all of the light emitting devices 10a through 10d to illuminate the subject with white light. Further, the spectral image generation means 33 generates, by using Formula (1), spectral estimation image SP from the endoscopic image P that has been obtained by illumination with white light.

Alternatively, the light source control means 50 may drive a combination of light emitting devices selected from the plurality of light emitting devices, the combination achieving the highest estimation accuracy. Specifically, the light source control means 50 stores, in advance, a combination of ON/OFF of the light emitting devices 10a through 10d, the combination achieving the highest spectral estimation accuracy when an observation region corresponding to each wavelength set, or a color patch that has substantially the same color as the observation region is illuminated with light by combining ON/OFF of each of the light emitting devices 10a through 10d. For example, when wavelength bands of a wavelength set overlap with a part of the wavelength bands of the light emitting devices 10a through 10d, as in wavelength set CH3 illustrated in Figure 3, the light source control means 50 controls ON/OFF of the light emitting devices 10a through 10d by using a combination of ON/OFF of each of the light emitting devices 10a through 10d that has been stored in advance.

As described above, the wavelength bands of light output from the light source unit 10 are selected based on the set wavelength sets CH1 through CH3. Therefore, it is possible to efficiently generate spectral estimation image SP that has high estimation accuracy. As in a conventional method, when a spectral image is obtained by using an optical filter, the structure of the apparatus tends to become complex. Further, when images are obtained, as separate frame images, by illumination with light having different wavelength bands from each other, and matrix operation is performed, the frame rate becomes lower, and efficient image diagnosis is impossible. In contrast, in the present invention, the wavelength bands of light output from the light source unit 10 are selected based on the wavelength sets CH1 through CH3, and the light source unit 10 is driven to obtain a single endoscopic image P by illumination with light of the selected wavelength bands. Further, spectral estimation image SP is obtained by performing matrix operation on the single endoscopic image P. Therefore, it is possible to generate the spectral estimation image SP from the endoscopic image P, which corresponds to one frame image, in simple apparatus structure that does not require an optical filter and the like. Hence, efficient image diagnosis is possible.

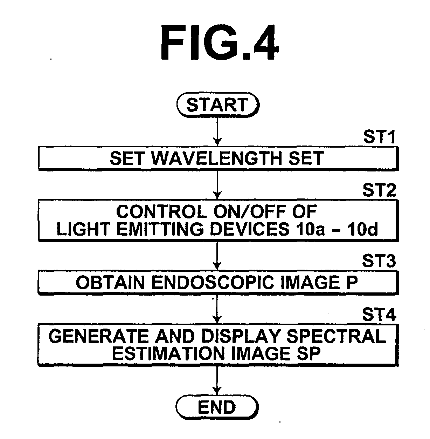

Figure 4 is a flow chart illustrating an endoscopic image obtainment method according to an embodiment of the present invention. The endoscopic image obtainment method will be described with reference to Figures 1 through 4. First, imaging is performed by the scope 20 inserted into the body cavity of a patient. At this time, the user selects one of wavelength sets CH1 through CH3 by using an input means 2, and the wavelength set setting means 40 sets the wavelength set for spectral estimation image SP (step ST1).

Further, the light source control means 50 drives the plurality of light emitting devices 10a through 10d by controlling ON/OFF of the plurality of light emitting devices 10a through 10d based on the wavelength set that has been set by the wavelength set setting means 40 (step ST2, please refer to Figure 3) . Further, the scope 20 images subject S illuminated with light of a predetermined wavelength band or bands to obtain endoscopic image P (step ST3). After then, the spectral image generation means 33 performs matrix operation on the endoscopic image P by using Formula (1) to generate spectral estimation image SP. Further, the display device 3 displays the spectral estimation image SP (step ST4).

According to the embodiments of the present invention, a plurality of wavelength bands to be represented by spectral estimation image SP are set as wavelength sets CH1 through CH3. Further, the light source unit 10 including the plurality of light emitting devices 10a through 10d, which output light of different wavelength bands from each other, is controlled. The light source unit 10 can output white light by driving the plurality of light emitting devices 10a through 10d. ON/OFF of each of the plurality of light emitting devices 10a through 10d is controlled based on the set wavelength set CH1 through CH3. Further, endoscopic image P is obtained by imaging the subject by the scope 20 while ON/OFF of each of the light emitting devices 10a through 10d is controlled. Further, matrix operation based on wavelength set CH1 through CH3 is performed on the endoscopic image P obtained by the scope 20 to generate spectral estimation image SP. Therefore, in the present invention, light of wavelength bands that are necessary to generate the spectral estimation image SP from the single endoscopic image P is output. Hence, the present invention can improve the estimation accuracy of the spectral estimation image SP, and efficiently generate the spectral estimation image SP.

Further, when the wavelength set setting means 40 sets a wavelength set by selecting one of the plurality of wavelength sets CH1 through CH3, which have been prepared in advance, and the light source unit 10 includes a plurality of light emitting devices corresponding to respective wavelength bands included in the plurality of wavelength sets CH1 through CH3, if the user selects an arbitrary wavelength set, the user can automatically drive-control the light emitting devices 10a through 10d based on the selected wavelength set. Therefore, it is possible to improve the estimation accuracy of the spectral estimation image SP, and to efficiently generate the spectral estimation image SP.

Further, when at least one of the wavelength bands included in the wavelength sets CH1 through CH3 overlaps with none of the wavelength bands of the light emitting devices 10a through 10d, if the light source control means 50 drives a combination of light emitting devices 10a through 10d, the combination achieving highest estimation accuracy, it is possible to maintain estimation accuracy of the spectral estimation image SP at a high level even if at least one of the wavelength bands included in the wavelength sets overlaps with none of the wavelength bands of the light emitting devices 10a through 10d.

The embodiments of the present invention are not limited to the embodiments described above. For example, a case in which the light source unit 10 includes four kinds of light emitting devices (LED) 10a through 10d was described. However, the number of the kinds of light emitting devices is not limited to four, and the number of the kinds may be greater than four. Further, a case in which each of the light emitting devices 10a through 10d is arranged at the leading end of the scope 20 was described as an example. Alternatively, the light emitting devices 10a through 10d may be provided in a separate unit, which is different from the scope 20, and light transmitted through a light guide, such as an optical fiber, may be output to the subject from the leading end of the scope 20.

## Claims

1. An endoscopic apparatus comprising:

   a light source unit (10) including a plurality of light emitting devices (10a through 10d) that output light of different wavelength bands from each other, the light source unit (10) being able to output white light by driving the plurality of light emitting devices (10a through 10d);
   a scope (20) that obtains an endoscopic image (P) by imaging a subject illuminated with light output from the light source unit (10);
   a wavelength set setting means (40) that sets a plurality of wavelength bands, as a wavelength set (CH1 through CH3);
   a light source control means (50) that *sets one of plural possible* combinations of the plurality of light emitting devices (10a through 10d) in ON and OFF states, based on the wavelength set (CH1 through CH3) that has been set by the wavelength set setting means (40); and
   a spectral image generation means (33) that generates a spectral estimation image (SP) by performing, based on the wavelength set (CH1 through CH3) that has been set by the wavelength set setting means (40), matrix operation on the endoscopic image (P) by using matrix *parameters* (M), and
   when at least one of the wavelength bands included in the wavelength set (CH1 through CH3) overlaps with none of the wavelength bands of the plurality of light emitting devices (10a through 10d), the light source control means (50) drives *all of the light emitting devices (10a through 10d) to illuminate the subject with white light,* or a portion of the light emitting devices (10a through 10d), which is stored in advance *as a combination achieving the highest estimation accuracy,* corresponding to the wavelength set of the spectral estimation image (SP) from among the plurality of the light emitting devices (10a through 10d).

2. An endoscopic apparatus, as defined in claim 1, wherein when each of the wavelength bands included in the wavelength set (CH1 through CH3) overlaps with at least one of the wavelength bands of the plurality of light emitting devices (10a through 10d), the light source control means (50) drives only the light emitting devices (10a through 10d) of the overlapping wavelength bands.

3. An endoscopic apparatus, as defined in claim 1 or 2, wherein the wavelength set setting means (40) sets the wavelength set (CH1 through CH3) by selecting the wavelength set (CH1 through CH3) from a plurality of wavelength sets (CH1 through CH3) that have been prepared in advance, and wherein the light source unit (10) includes the plurality of light emitting devices (10a through 10d) corresponding to respective wavelength bands included in the plurality of wavelength sets (CH1 through CH3).

4. An endoscopic image obtainment method comprising the steps of:

setting a plurality of wavelength bands, as a wavelength set (CH1 through CH3);
controlling a light source unit (10) including a plurality of light emitting devices (10a through 10d) that output light of different wavelength bands from each other, the light source unit (10) being able to output white light by driving the plurality of light emitting devices (10a through 10d), by *setting one of plural possible combinations of ON/OFF* states of each of the plurality of light emitting devices (10a through 10d) based on the set wavelength set (CH1 through CH3); and
generating a spectral estimation image (SP) by performing, based on the set wavelength set (CH1 through CH3), matrix operation on an endoscopic image (P) that has been obtained by a scope (20) *by using matrix parameters,* while ON/OFF of each of the plurality of light emitting devices (10a through 10d) is controlled, and when at least one of the wavelength bands included in the wavelength set (CH1 through CH3) overlaps with none of the wavelength bands of the plurality of light emitting devices (10a through 10d), driving *all of the light emitting devices (10a through 10d) to illuminate the subject with white light, or* a portion of the light emitting devices (10a through 10d), which is stored in advance *as a combination achieving the highests estimation accuracy,* corresponding to the wavelength set of the spectral estimation image (SP) from among the plurality of the light emitting devices (10a through 10d).

**Patentansprüche**

1. Endoskopvorrichtung, umfassend:

eine Lichtquelleneinheit (10) mit einer Mehrzahl von Lichtemissionsbauelementen (10a bis 10d), die Licht mit voneinander verschiedenen Wellenlängenbändern ausgeben, wobei die Lichtquelleneinheit (10) in der Lage ist, durch Treiben der mehreren Lichtemissionsbauelemente (10a bis 10d) weißes Licht abzugeben;
ein Endoskop (20), welches durch Abbilden eines mit von der Lichtquelleneinheit (10) ausgegebenem Licht beleuchteten Subjekt ein Endoskopbild (P) erhält;
eine Wellenlängensatz-Einstelleinrichtung (40), die eine Mehrzahl von Wellenlängenbändern als einen Wellenlängensatz (CH1 bis CH3) einstellt;
eine Lichtquellen-Steuereinrichtung (50), die eine von mehreren möglichen Kombinationen der mehreren Lichtemissionsbauelemente (10a bis 10d) in EIN- und AUS-Zustände versetzt, basierend auf dem Wellenlängensatz (CH1 bis CH3), der von der Wellenlängensatz-Einstelleinrichtung (40) eingestellt wurde; und
eine Spektralbild-Erzeugungseinrichtung (33), die ein Abschätz-Spektralbild (SP) dadurch erzeugt, dass sie basierend auf dem von der Wellenlängensatz-Einstelleinrichtung (40) eingestellten Wellenlängensatz (CH1 bis CH3) bezüglich des Endoskopbilds (P) unter Verwendung von Matrixparametern (M) eine Matrixoperation ausführt, und
wenn mindestens eines der in dem Wellenlängensatz (CH1 bis CH3) enthaltenen Wellenlängenbänder sich mit keinem der Wellenlängenbänder der mehreren Lichtemissionsbauelemente (10a bis 10d) überlappt, die Lichtquellen-Steuereinrichtung (50) sämtliche Lichtemissionsbauelemente (10a bis 10d) zum Beleuchten des Subjekts mit weißem Licht treibt, oder einen Teil der Lichtemissionsbauelemente (10a bis 10d), der vorab als die höchste Abschätzgenauigkeit erreichende Kombination eingestellt wurde, entsprechend dem Wellenlängensatz des Abschätz-Spektralbilds (SP) unter den mehreren Lichtemissionsbauelementen (10a bis 10d) treibt.

2. Endoskopvorrichtung nach Anspruch 1, bei der, wenn jedes der Wellenlängenbänder in dem Wellenlängensatz

**EP 2 258 253 B1**

(CH1 bis CH3) sich mit mindestens einem der Wellenlängenbänder der mehreren Lichtemissionsbauelemente (10a bis 10d) überlappt, die Lichtquellen-Steuereinrichtung (50) nur die Lichtemissionsbauelemente (10a bis 10d) der überlappenden Wellenlängenbänder treibt.

3. Endoskopvorrichtung nach Anspruch 1 oder 2, bei der die Wellenlängensatz-Einstelleinrichtung (40) den Wellenlängensatz (CH1 bis CH3) dadurch einstellt, dass sie den Wellenlängensatz (CH1 bis CH3) aus einer Mehrzahl von Wellenlängensätzen (CH1 bis CH3) auswählt, die vorab bereitgestellt wurden, und wobei die Lichtquelleneinheit (10) die mehreren Lichtemissionsbauelemente (10a bis 10d) entsprechend den mehreren, in den mehreren Wellenlängensätzen (CH1 bis CH3) enthaltenen Wellenlängenbändern enthält.

4. Endoskopbild-Gewinnungsverfahren, umfassend folgende Schritte:

Einstellen einer Mehrzahl von Wellenlängenbändern in Form eines Wellenlängensatzes (CH1 bis CH3);
Steuern einer Lichtquelleneinheit (10), die mehrere Lichtemissionsbauelemente (10a bis 10d) enthält, die Licht mit voneinander verschiedenen Wellenlängenbändern ausgeben, wobei die Lichtquelleneinheit (10) in der Lage ist, durch Treiben der mehreren Lichtemissionsbauelemente (10a bis 10d) dadurch weißes Licht abzugeben, dass eine von mehreren möglichen Kombinationen von EIN-/AUS-Zuständen jedes der mehreren Lichtemissionsbauelemente (10a bis 10d) basierend auf dem eingestellten Wellenlängensatz (CH1 bis CH3) eingestellt wird; und
Erzeugen eines Abschätz-Spektralbilds (SP), indem basierend auf dem eingestellten Wellenlängensatz (CH1 bis CH3) eine Matrixoperation an dem Endoskopbild (P), das von dem Endoskop (20) gewonnen wurde, unter Verwendung von Matrixparametern ausgeführt wird, während der EIN-/AUS-Zustand jedes der mehreren Lichtemissionsbauelemente (10a bis 10d) gesteuert wird, und
wenn mindestens eines der in dem Wellenlängensatz (CH1 bis CH3) enthaltenen Wellenlängenbänder sich mit keinem der Wellenlängenbänder der mehreren Lichtemissionsbauelemente (10a bis 10d) überlappt, sämtliche der Lichtemissionsbauelemente (10a bis 10d) zum Beleuchten des Subjekts mit weißem Licht getrieben werden, oder ein Teil der Lichtemissionsbauelemente (10a bis 10d) getrieben werden, der vorab gespeichert wurde als Kombination zur Erzielung der höchsten Abschätzgenauigkeit, entsprechend dem Wellenlängensatz des Abschätz-Spektralbilds (SP) unter den mehreren Lichtemissionsbauelementen (10a bis 10d).

**Revendications**

1. Appareil endoscopique comprenant :

une unité de source de lumière (10) incluant une pluralité de dispositifs émetteurs de lumière (10a à 10d) qui fournissent en sortie de la lumière dans des bandes de longueurs d'ondes différentes les unes des autres, l'unité de source de lumière (10) étant capable de fournir en sortie de la lumière blanche en commandant la pluralité de dispositifs émetteurs de lumière (10a à 10d) ;
un dispositif de visualisation (20) permettant d'obtenir une image endoscopique (P) par formation d'image d'un sujet éclairé par la lumière fournie en sortie par l'unité de source de lumière (10) ;
un moyen de réglage d'ensemble de longueurs d'ondes (40) qui règle une pluralité de bandes de longueurs d'ondes, comme ensemble de longueurs d'onde (CH1 à CH3) ;
un moyen de commande de source de lumière (50) qui règle une combinaison parmi plusieurs combinaisons possibles de la pluralité de dispositifs émetteurs de lumière (10a à 10d) dans des états ACTIVÉ et DÉSACTIVÉ, sur la base de l'ensemble de longueurs d'ondes (CH1 à CH3) ayant été réglé par le moyen de réglage d'ensemble de longueurs d'ondes (40) ; et
un moyen de génération d'image spectrale (33) qui génère une image d'estimation spectrale (SP) en effectuant, sur la base de l'ensemble de longueurs d'ondes (CH1 à CH3) ayant été réglé par le moyen de réglage d'ensemble de longueurs d'ondes (40), une opération matricielle sur l'image endoscopique (P) en utilisant des paramètres de matrice (M), et
lorsqu'au moins l'une des bandes de longueurs d'ondes incluses dans l'ensemble de longueurs d'ondes (CH1 à CH3) n'est superposée avec aucune des bandes de longueurs d'ondes de la pluralité de dispositifs émetteurs de lumière (10a à 10d), le moyen de commande de source de lumière (50) commande tous les dispositifs émetteurs de lumière (10a à 10d) pour éclairer le sujet avec de la lumière blanche, ou une partie des dispositifs émetteurs de lumière (10a à 10d) qui est stockée à l'avance en tant que combinaison permettant d'obtenir la plus grande précision d'estimation, correspondant à l'ensemble de longueurs d'ondes de l'image d'estimation spectrale (SP) parmi la pluralité de dispositifs émetteurs de lumière (10a à 10d).

**2.** Appareil endoscopique selon la revendication 1, dans lequel, lorsque chacune des bandes de longueurs d'ondes incluses dans l'ensemble de longueurs d'ondes (CH1 à CH3) est superposée avec au moins l'une des bandes de longueurs d'ondes de la pluralité de dispositifs émetteurs de lumière (10a à 10d), le moyen de commande de source de lumière (50) commande uniquement les dispositifs émetteurs de lumière (10a à 10d) des bandes de longueurs d'ondes en superposition.

**3.** Appareil endoscopique selon la revendication 1 ou 2, dans lequel le moyen de réglage d'ensemble de longueurs d'ondes (40) règle l'ensemble de longueurs d'ondes (CH1 à CH3) en sélectionnant l'ensemble de longueurs d'ondes (CH1 à CH3) parmi une pluralité d'ensembles de longueurs d'ondes (CH1 à CH3) ayant été préparés à l'avance, et dans lequel l'unité de source de lumière (10) comporte la pluralité de dispositifs émetteurs de lumière (10a à 10d) correspondant aux bandes de longueurs d'ondes respectives incluses dans la pluralité d'ensembles de longueurs d'ondes (CH1 à CH3).

**4.** Procédé d'obtention d'image endoscopique comprenant les étapes consistant à :

régler une pluralité de bandes de longueurs d'ondes, comme ensemble de longueurs d'onde (CH1 à CH3) ;
commander une unité de source de lumière (10) incluant une pluralité de dispositifs émetteurs de lumière (10a à 10d) qui fournissent en sortie de la lumière dans des bandes de longueurs d'ondes différentes les unes des autres, l'unité de source de lumière (10) étant capable de fournir en sortie de la lumière blanche en commandant la pluralité de dispositifs émetteurs de lumière (10a à 10d), par réglage d'une combinaison parmi plusieurs combinaisons possibles d'états ACTIVÉ/DÉSACTIVÉ de chaque dispositif de la pluralité de dispositifs émetteurs de lumière (10a à 10d), sur la base de l'ensemble de longueurs d'ondes (CH1 à CH3) réglé ; et
générer une image d'estimation spectrale (SP) en effectuant, sur la base de l'ensemble de longueurs d'ondes (CH1 à CH3) réglé, une opération matricielle sur une image endoscopique (P) ayant été obtenue par un dispositif de visualisation (20), en utilisant des paramètres de matrice, pendant l'état ACTIVÉ/DÉSACTIVÉ de chaque dispositif de la pluralité de dispositifs émetteurs de lumière (10a à 10d), et
lorsqu'au moins l'une des bandes de longueurs d'ondes incluses dans l'ensemble de longueurs d'ondes (CH1 à CH3) n'est superposée avec aucune des bandes de longueurs d'ondes de la pluralité de dispositifs émetteurs de lumière (10a à 10d), commander tous les dispositifs émetteurs de lumière (10a à 10d) pour éclairer le sujet avec de la lumière blanche, ou une partie des dispositifs émetteurs de lumière (10a à 10d) qui est stockée à l'avance en tant que combinaison permettant d'obtenir la plus grande précision d'estimation, correspondant à l'ensemble de longueurs d'ondes de l'image d'estimation spectrale (SP) parmi la pluralité de dispositifs émetteurs de lumière (10a à 10d).

# FIG.1

# FIG.2

DB

| PARAMETER (WAVELENGTH) | $M_{j0}$ | $M_{j1}$ | $M_{j2}$ |
|---|---|---|---|
| p1 | 0.000083 | −0.00188 | 0.003592 |
| ⋮ | ⋮ | ⋮ | ⋮ |
| p18 | −0.00115 | 0.000569 | 0.003325 |
| p19 | −0.00118 | 0.001149 | 0.002771 |
| p20 | −0.00118 | 0.001731 | 0.0022 |
| p21 | −0.00119 | 0.002346 | 0.0016 |
| p22 | −0.00119 | 0.00298 | 0.000983 |
| p23 | −0.00119 | 0.003633 | 0.000352 |
| ⋮ | ⋮ | ⋮ | ⋮ |
| p43 | 0.003236 | 0.001377 | −0.00159 |
| p44 | 0.003656 | 0.000671 | −0.00126 |
| p45 | 0.004022 | 0.000068 | −0.00097 |
| p46 | 0.004342 | −0.00046 | −0.00073 |
| p47 | 0.00459 | −0.00088 | −0.00051 |
| p48 | 0.004779 | −0.00121 | −0.00034 |
| p49 | 0.004922 | −0.00148 | −0.00018 |
| p50 | 0.005048 | −0.00172 | −0.000036 |
| p51 | 0.005152 | −0.00192 | 0.000088 |
| p52 | 0.005215 | −0.00207 | 0.000217 |
| ⋮ | ⋮ | ⋮ | ⋮ |
| p61 | 0.00548 | −0.00229 | 0.00453 |

# FIG.3

| | WAVELENGTH SET | | | LIGHT EMITTING DEVICE 10a (470nm) | LIGHT EMITTING DEVICE 10b (500nm) | LIGHT EMITTING DEVICE 10c (540nm) | LIGHT EMITTING DEVICE 10d (620nm) |
|---|---|---|---|---|---|---|---|
| | R COMPONENT | G COMPONENT | B COMPONENT | | | | |
| CH1 | 550nm | 500nm | 470nm | ON | ON | ON | OFF |
| CH2 | 525nm | 495nm | 495nm | ON | ON | ON | ON |
| CH3 | 540nm | 415nm | 415nm | ON | OFF | ON | ON |

EP 2 258 253 B1

# FIG.4

```
                  ( START )
                       │
                       ▼            ST1
     ┌───────────────────────────────────┐
     │        SET WAVELENGTH SET          │
     └───────────────────────────────────┘
                       │
                       ▼            ST2
     ┌───────────────────────────────────┐
     │        CONTROL ON/OFF OF           │
     │  LIGHT EMITTING DEVICES 10a - 10d  │
     └───────────────────────────────────┘
                       │
                       ▼            ST3
     ┌───────────────────────────────────┐
     │     OBTAIN ENDOSCOPIC IMAGE P      │
     └───────────────────────────────────┘
                       │
                       ▼            ST4
     ┌───────────────────────────────────┐
     │  GENERATE AND DISPLAY SPECTRAL     │
     │     ESTIMATION IMAGE SP            │
     └───────────────────────────────────┘
                       │
                       ▼
                  (  END  )
```

13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006166940 A
- JP 2007264537 A
- JP 2003093336 A